Europäisches Patentamt

European Patent Office

Office européen des brevets

(11) Veröffentlichungsnummer: **0 455 928 A1**

# (12) EUROPÄISCHE PATENTANMELDUNG

(21) Anmeldenummer: 90890349.5

(22) Anmeldetag: 20.12.90

(51) Int. Cl.5: **C12N 9/24**, C12N 1/14

(30) Priorität: 08.05.90 AT 1036/90

(43) Veröffentlichungstag der Anmeldung:
**13.11.91 Patentblatt 91/46**

(84) Benannte Vertragsstaaten:
**AT BE CH DE DK ES FR GB GR IT LI LU NL SE**

(71) Anmelder: **LENZING AKTIENGESELLSCHAFT**
**Werkstrasse 1**
**A-4860 Lenzing(AT)**

(72) Erfinder: **Gamerith, Gernot, Dipl.-Ing. Dr.**
**Feldgasse 14/1**
**A-4840 Vöcklabruck(AT)**
Erfinder: **Emeder, Rudolf**
**Kemating 10**
**A-4863 Seewalchen(AT)**
Erfinder: **Groicher, Rene**
**Stelzhammerstrasse 34**
**A-4850 Timelkam(AT)**

(74) Vertreter: **Müllner, Erwin, Dr. et al**
**Patentanwälte, Dr. Erwin Müllner, Dipl.-Ing.**
**Werner Katschinka, Dr. Martin Müllner,**
**Postfach 159, Weihburggasse 9**
**A-1010 Wien(AT)**

(54) Verfahren zur Herstellung einer Xylanase-reichen und Cellulase-armen Enzymlösung.

(57) Um eine Xylanase-reiche Enzymlösung, die möglichst wenig Cellulase enthält, kostengünstig herzustellen, wird vorgeschlagen, bekannte, Xylanase produzierende Mikroorganismen auf Abfallauge aus dem Viskoseverfahren oder auf Abwasser, das bei einer Kunstfaserzellstoffbleiche mit alkalischer Extraktion, die durch Sauerstoff und/oder Wasserstoffperoxid unterstützt sein kann, anfällt, zu züchten. Es ist zweckmäßig, vorher die Abfallauge bzw. das Abwasser zu sterilisieren, zu neutralisieren und mit Stickstoffquellen, beispeilsweise Pepton, Klärschlamm und/oder Harnstoff, zu versetzen. Die Anzucht kann mit Stämmen von Trichoderma, Aspergillus oder Schizophyllum erfolgen. Während des Züchtens entsteht die gewünschte Enzymlösung, die durch Abtrennen der Biomasse isoliert werden kann.

EP 0 455 928 A1

Die vorliegende Erfindung betrifft ein Verfahren zur Herstellung einer Xylanase-reichen und Cellulase-armen Enzymlösung durch Züchtung herkömmlicher, Xylanase produzierender Mikroorganismen und anschließender Abtrennung der Biomasse.

Xylanase kann in der Zellstoffindustrie prinzipiell als Bleichchemikalie oder Veredelungschemikalie eingesetzt werden. Für einen wirtschafltichen Einsatz müssen dabei aber zwei Bedingungen erfüllt werden: einerseits muß die Enzymlösung im Hinblick auf Cellulasen relativ rein sein, weil sonst auch die Cellulose abgebaut oder geschädigt wird; andererseits ist ein für Enzymprozesse ungewöhnlich billiges Herstellungsverfahren notwendig, weil sonst infolge der großen benötigten Enzymmengen die Rentabilität der gesamten Zellstoffproduktion nicht mehr gegeben ist.

Es ist bekannt, daß viele in der Natur vorkommende Mikroorganismen in der Lage sind, Enzyme zu bilden, welche die verschiedenen Holzkomponenten Cellulose, Hemicellulose und Lignin abbauen können. Die Herstellung von Xylanase kann daher durch Anzucht derartiger Mikroorganismen auf einem Substrat, welches grundsätzlich eine Kohlenstoffquelle, Stickstoffquellen und bestimmte Salze enthalten muß, erfolgen. Als Xylanaseproduzenten sind u.a. beschrieben:

Trichoderma reseei, T. harzianum, T. viride
Aspergillus niger, A. fumigatus, A. foetidus
Schizophyllum commune
Bacillus subtilis
Cryptococcus albidus
Saccharomonospora viridis
Aureobasidium pullulans
Humicola sp.
Phaenerochaete chrysosporium
Streptomyces sp.
Solche Stämme sind bei ATCC erhältlich:
z.B. ATCC Nr.56 765 (Trichoderma reesei Rutgen C30)
ATCC Nr.26 921 (Trichoderma reesei QM 9440)
ATCC Nr.46 324 (Aspergillus fumigatus)
ATCC Nr.38 548 (Schizophyllum commune)

Allerdings produzieren alle Mikroorganismen Enzymgemische, die mehr als nur eine der Komponenten von lignocellulosischem Material abbauen. Die Gegenwart von Cellulase ist jedoch manches Mal unerwünscht.

Folgende Möglichkeiten sind bisher untersucht worden, um die Reinheit der gebildeten Xylanase zu erhöhen, sodaß sie Weniger Cellulase enthält:

a) Genetische Veränderung von Mikroorganismen durch Einbau von Xylanasegenen in das Bakterium Escherichia coli.
Lit: Gene 26(1983), 59-65
Nachteil dieses Verfahrens sind die sehr geringen Enzymausbeuten.

b) Beeinflussung des gebildeten Enzymsspektrums durch Nährlösungskomponenten: die Anwesenheit von reinem Xylan als Kohlenstoffquelle im Nährmedium ermöglicht eine Verschiebung des Verhältnisses zwischen Xylanase und Cellulase zugunsten der Xylanase.
Lit: Appl. Environ.Microbiol. 52(1986) 1026-1030
Nachteil dieser Verfahren ist die nicht ausreichende Verfügbarkeit von Xylanquellen ausreichender Reinheit im großtechnischen Maßstab bzw. der zu hohe Preis für Xylan als Kohlenstoffquelle.

c) Selektive Induktion der Xylanasebildung durch geeignete Induktoren: wird eine andere Kohlenstoffquelle als Xylan eingesetzt, bewirkt die zusätzliche Zugabe von Xylan ebenfalls eine Verschiebung des Verhältnisses von Xylanase zu Cellulase zugunsten von Xylanase. Noch wirkungsvollere Induktoren sind Xylobiose und insbesondere $\beta$-Methylxylosid.
Lit: Appl. Microbiol Biotechn. 30 (1989) 5-10
Für technische Prozesse werden als Rohstoffe für die Enzymgewinnung vorwiegend landwirtschaftliche Abfälle, wie z.B. Maiskolben, Weizenkleie, Stroh, Bagasse oder Zellstoff unter Verwendung einer der oben genannten Mikroorganismen ins Auge gefaßt, s.z.B. SU 658 168 (1979).

d) Trennung von Xylanase und Cellulase: in der US-PS 4 725 544 (1988) ist die Trennung von Cellulase und Xylanase durch Ultrafiltration beschrieben. Nachteilig sind die damit verbundenen hohen Kosten, die solch ein Produkt für eine breite Anwendung in der Zellstoffindustrie zu teuer machen.

Es ist Aufgabe der vorliegenden Erfindung, ein Verfahren zur Herstellung einer Xylanase-reichen und Cellulase-armen Enzymlösung zu schaffen, das kostengünstiger und selektiver als die bekannten Verfahren ist. Weiters soll durch dieses Verfahren die Abwasserbelastung durch Prozeßabwässer, die in der Zellstoffindustrie anfallen, verringert werden.

Diese Aufgaben werden durch ein Verfahren der eingangs genannten Art erfindungsgemäß dadurch gelöst, daß die Mikroorganismen auf neutralisierter, mit Nährsalzen versetzter Abfallauge aus dem Viskosenverfahren oder auf neutralisiertem, mit Nährsalzen versetztem Abwasser, das bei einer Kunstfaserzellstoffbleiche mit alkalischer Extraktion, die gegebenenfalls durch Sauerstoff und/oder Wasserstoffperoxid unterstützt wird, anfällt, gezüchtet werden.

Die Mikroorganismen werden etwa 2 bis 10 Tage auf der Abfallauge bzw. dem Abwasser gezüchtet. Während dieser Zeit wird das gewünschte Enzym produziert. Nach Beendigung der Fermentation wird die Biomasse abfiltriert oder abzentrifugiert. Das Filtrat oder Zentrifugat kann ohne weitere Aufarbeitung zur Behandlung von Zellstoff in Hinblick auf Bleiche oder Veredelung eingesetzt werden. Selbstverständlich können aber vorher auch noch herkömmliche Reinigungsverfahren durchgeführt werden.

Es ist zweckmäßig, wenn die Abfallauge bzw. das Abwasser sterilisiert wird, bevor die Mikroorganismen zugesetzt werden; dadurch wird sichergestellt, daß nur die gewünschten Mikroorganismen gezüchtet werden und keine unerwünschten Verunreinigungen durch andere Mikroorganismen gebildet werden.

Vorzugsweise wird der Abfallauge bzw. dem Abwasser eine Stickstoffquelle, vorzugsweise Pepton, Klärschlamm und/oder Harnstoff, zugesetzt. Dies wird immer dann notwendig sein, wenn das Abwasser bzw. die Abfallauge selbst keine Stickstoffquellen enthalten.

Nach einer weiteren Ausgestaltung der Erfindung werden als Mikroorganismen Stämme von Trichoderma, Aspergillus oder Schizophyllum verwendet.

Es wird bevorzugt, daß die Abfallauge bzw. das Abwasser auf einenpH-Wert von 4 bis 7, vorzugsweise von 4 bis 5, gebracht wird. Nach einem weiteren Merkmal der Erfindung wird die Enzymlösung vom Rückstand abfiltriert.

Infolge der Verwendung von Abfallauge bzw. Abwasser wird das erfindungsgemäße Verfahren sehr kostengünstig, weil ja für das eigentliche Nährmittel keinerlei Kosten entstehen.

Beim Viscoseverfahren führt man gereinigte Cellulose zunächst durch Behandeln mit Natronlauge in Alkalicellulose über (Alkalisierung). Nach Abpressen der überschüssigen Natronlauge wird die Alkalicellulose zerfasert und dann, falls der Polymerisationsgrad der Cellulose gesenkt werden soll, der sog. Vorreife unterworfen, die einen oxydativen Abbau darstellt. Die so vorbehandelte Alkalicellulose läßt man mit Schwefelkohlenstoff reagieren, wobei das Natriumsalz der Cellulosexanthogensäure entsteht, das sich in Wasser oder Lauge zur sogenannten Viscose auflöst. Für die vollständige und einwandfreie Lösung ist ein hinreichend hoher Substitutionsgrad erwünscht, während für das Spinnen, je nach Verfahren, meist ein geringerer Substitutionsgrad erforderlich ist. Man überläßt daher die Viscose der sogenannten Nachreife, in deren Verlauf ein Teil der Xanthatgruppen wieder abgespalten wird.

Die filtrierte und entlüftete Spinnlösung wird zu Fäden versponnen, zu Filmen, Kapseln und Schläuchen oder auch zu Viscoseschwämmen und -schwammtüchern verarbeitet.

Die zur Herstellung der Alkalicellulose zugesetzte NaOH hat etwa 17 bis 19 Masse-%. Die fertige Alkalicellulose wird auf etwa das dreifache Gewicht der Cellulose (Preßfaktor 1:2,5 bis 1:3,3) abgepreßt, so daß eine Alkalicellulose resultiert, die normalerweise 14,8-16% NaOH und 30-36% Cellulose enthält. Die Gelblauge enthält 1-2% weniger NaOH als die frische Tauchlauge sowie Hemicellulose. Sie kann nach Abfiltrieren der Cellulosefasern meist direkt zum Verdünnen der Frischlauge verwendet werden. Die in der Schlußphase der Abpressung anfallende Preßlauge, die bis zu 6% Hemicellulose enthalten kann, muß dagegen zuerst durch Dialyse, wobei eine 4-8%-ige Regeneratlauge anfällt, regeneriert werden.

Überraschend ist die Tatsache, daß Abfallauge der Dialyse der Viskosefabrikation nach Neutralisation und Zugabe der auch sonst üblichen Nährmittel, jedoch keiner zusätzlichen Kohlenstoffquelle als Nährmedium eine selektive Herstellung von Xylanase erlaubt. Dies ist insofern überraschend, als die Abfallauge nach der Neutralisation einerseits einen für viele Mikroorganismen unverträglich hohen Gehalt an Natriumsulfat aufweist (ca. 35 g/l), anderseits auch als Kohlenstoffquelle neben dem Xylan noch eine Reihe von Hydroxycarbonsäuren enthält, deren Auswirkung auf den Stoffwechsel von Mikroorganismen nicht vorhersehbar ist.

Ähnliches gilt auch für das Abwasser, das bei einer Bleiche von Kunstfaserzellstoff (z.B. von Buchenholz) mit alkalischer Extraktion, die durch $O_2$ oder $H_2O_2$ unterstützt sein kann, anfällt. Es ist beim Bleichen von Zellstoff bereits bekannt, chlorfrei zu arbeiten, um die Umwelt dadurch zu schonen. Eine Möglichkeit für eine chlorfreie Bleichstufe ist eine OPE-Stufe, d.h. man führt eine alkalische Extraktion unter Zugabe von Sauerstoff und Wasserstoffperoxid durch. Dazu versetzt man Rohzellstoff mit Natronlauge und $H_2O_2$ bei etwas erhöhter Temperatur. Danach wird $O_2$ zugeführt, wobei Rühren zweckmäßig sein kann. Dieses Gemisch wird dann für 2 bis 3 Stunden in einem Bleichturm reagieren gelassen. Danach wird der Zellstoff durch Filter oder Pressen abgetrennt. Das dabei anfallende Abwasser enthält keine monomeren oder polymeren Zuckermoleküle; es ist deshalb erstaunlich, daß sich auch dieses Abwasser für eine Enzymbildung eignet.

Überraschenderweise zeigte es sich auch, daß die auf solchen Substraten angezüchteten Enzymlösungen ein günstiges Verhältnis zwischen Xylanase und Cellulase, d.h. nur eine geringe Restaktivität an Cellulase, aufweisen.Die schädliche Wirkung auf Cellulose ist bei Enzymlösungen, die durch Züchtung eines Mikroorganismus auf Abfallauge erhalten wird, bedeutend geringer als bei Enzymlösungen, die bei Anzucht des Mikroorganismus auf reinem Xylan erhalten wird. Dieser Umstand macht eine Aufarbeitung über die Filtration des Pilzmycels hinaus unnötig, es kann unmittelbar die vom Pilz befreite Enzymlösung mit oder ohne zusätzliche Eindampfung für die Zellstoffbehandlung verwendet werden.

Gegenüber jenen Verfahren, die auf die Herstellung möglichst reiner Xylanasen abzielen, ist somit der Vorteil des erfindungsgemäßen Verfahrens in den günstigen Rohstoffkosten zu sehen, gegenüber jenen Verfahren, bei denen eine Trennung von Xylanasen und Cellulasen notwendig ist, besteht der Vorteil in der von vornherein geringeren Cellulasemenge, welche eine weitere Trennung zumindest erleichtert, wenn nicht überflüssig macht.

Die folgenden Beispiele sollen das Verfahren näher beschreiben:

Beispiel 1:

300 ml einer vorbehandelten Abfallauge werden mit 0,6% Pepton, 0,05% Harnstoff, 0,15% Ammonsulfat, 0,05% Magnesiumsulfat und 1% KH$_2$PO$_4$ versehen und im Autoklaven 20 min bei 120° sterilisiert. Danach wird diese Lösung mit ca. 20 ml einer 4 - 5 (1d = 1 Tag) alten Vorkultur von Trichoderma reesei Rut C 30 auf 2% Xylan beimpft. Die Suspension wird bei 30° und einer Drehzahl von 110 min im Schüttelschrank über einen Zeitraum von 8 d inkubiert. Danach wird der Feststoff abfiltriert. Das Filtrat enthält 14 International Units (IU) an Xylanase und 0,24 IU an β-Xylosidase je ml.

Beispiel 2:

300 ml einer Nährlösung wie in Beispiel 1 werden mit 20 ml einer Vorkultur von Aspergillus fumigatus beimpft, dann wird wie im Beispiel 1 verfahren. Nach 96 h enthält das Filtrat 5,4 IU an Xylanase je ml.

Beispiel 3:

300 ml einer Nährlösung wie in Beispiel 1 werden mit einer Vorkultur von Trichoderma reesei QM 9414 beimpft und wie oben inkubiert. Nach 90,5 h enthält das Filtrat 36 IU an Xylanase und 0,24 IU an β-Xylosidase je ml.

Beispiel 4:

300 ml einer vorbehandelten Abfallauge werden versetzt mit 0,1% KH$_2$PO$_4$, 0,05% MgSO$_4$, 0,05% KCl und 0,6% Peptron und anschließend sterilisiert. Die Beimpfung erfolgt mit 20 ml einer Vorkultur von Schizophyllum commune, und die Anzucht erfolgt wie im Beispiel 1. Nach 10 d enthält das Filtrat 7 IU an Xylanase und 0,1 IU an Filterpapieraktivität (FPU) je ml.

Beispiel 5:

300 ml eines Abwassers der OPE-Stufe wird mit den in Beispiel 1 genannten Komponenten ergänzt und zur Anzucht von Trichoderma reesei Rut C 30 verwendet. Nach 93,5 h enthält das Filtrat 11 IU an Xylanase und 0,19 IU an Carboxymethylcellulase je ml.

Beispiel 6:

300 ml der gleichen Nährlösung wie in Beispiel 5 wird mit Trichoderma reesei QM 9414 beimpft und entsprechend obiger Beispiele inkubiert. Nach 90 h enthält das Filtrat 9,5 IU an Xylanase und 0,9 IU an Carboxymethylcellulase.

Beispiel 7:

7 l einer Nährlösung gemäß Beispiel 1 wird in einem Laborfermenter für 1 h bei 120° sterilisiert und anschließend mit 300 ml einer Vorkultur von Trichoderma reesei Rut C 30 beimpft. Die Kultivation erfolgt bei 50% Sauerstoffsättigung, 600 min$^{-1}$ und einem kopstanten pH von 5,5 über einen Zeitraum von 45 h. Nach dieser Zeit enthält das Filtrat der Fermentationsbrühe 60 IU an Xylanase je ml.

Beispiel 8:

300 ml einer vorbehandelten Abfallauge werden mit 0,1 % KH$_2$PO$_4$, 0,05 % MgSO$_4$, 0,05 % KCl und 3 % getrocknetem, pulverisiertem Klärschlamm versehen und anschließend sterilisiert. Die Beimpfung erfolgt mit 20 ml einer Vorkultur von Trichoderma reesei Rut C 30, und die Anzucht erfolgt wie in Beispiel 1.

Nach 8 Tagen enthält das Filtrat der Brühe 21 IU an Xylanase und 1,1 IU an Carboxymethylcellulase je ml.

**Patentansprüche**

1. Verfahren zur Herstellung einer Xylanase-reichen und Cellulase-armen Enzymlösung durch Züchtung herkömmlicher, Xylanase produzierender Mikroorganismen und anschließender Abtrennung der Biomasse, dadurch gekennzeichnet, daß die Mikroorganismen auf neutralisierter, mit Nährsalzen versetzter Abfallauge aus dem Viskoseverfahren oder auf neutralisiertem, mit Nährsalzen versetztem Abwasser, das bei einer Kunstfaserzellstoffbleiche mit alkalischer Extraktion, die gegebenenfalls durch Sauerstoff und/oder Wasserstoffperoxid unterstützt wird, anfällt, gezüchtet werden.

2. Verfahren nach Anspruch 1, dadurch gekennzeichnet, daß die Abfallauge bzw. das Abwasser sterilisiert wird, bevor die Mikroorganismen zugesetzt werden.

3. Verfahren nach Anspruch 1 oder 2, dadurch gekennzeichnet, daß der Abfallauge bzw. dem Abwasser eine Stickstoffquelle, vorzugsweise Pepton, Klärschlamm und/oder Harnstoff, zugesetzt wird.

4. Verfahren nach einem der Ansprüche 1 bis 3, dadurch gekennzeichnet, daß als Mikroorganismen Stämme von Trichoderma, Aspergillus oder Schizophyllum verwendet werden.

5. Verfahren nach einem der Ansprüche 1 bis 4, dadurch gekennzeichnet, daß die Abfallauge bzw. das Abwasser auf einen pH-Wert von 4 bis 7, vorzugsweise von 4 bis 5, gebracht wird.

6. Verfahren nach einem der Ansprüche 1 bis 5, dadurch gekennzeichnet, daß die Enzymlösung vom Rückstand abfiltriert wird.

# EUROPÄISCHER RECHERCHENBERICHT

**Europäisches Patentamt**

Nummer der Anmeldung

**EP 90 89 0349**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | US-A-4 725 544   (LARRY U.TAN ET AL.)<br>* Zusammenfassung *<br>— — — | 4,6 | C 12 N 9/24<br>C 12 N 1/14 |
| D,A | APPLIED MICROBIOLOGY AND BIOTECHNOLOGY vol. 30, 1989, BERLIN DE Seiten 5 - 10; M.J.Bailey et al.: "Production of xylanolytic enzymes by strains of Aspergillus"<br>* das ganze Dokument *<br>— — — | 2-6 | |
| A | CHEMICAL ABSTRACTS, vol. 105, no. 21, 24 November 1986 Columbus, Ohio, USA J.Hosokawa et al.: "Screening of microorganisms for cell mass production from solvolysis pulping<br>&Hakko Kogaku Kaishi,1986,64,5,443446 Seite 592; ref. no. 189438Z<br>* Zusammenfassung *<br>— — — — — | 1,4 | |

**RECHERCHIERTE SACHGEBIETE (Int. Cl.5)**

C 12 N 9
C 12 N 1

Der vorliegende Recherchenbericht wurde für alle Patentansprüche erstellt

| Recherchenort | Abschlußdatum der Recherche | Prüfer |
|---|---|---|
| Berlin | 08 Juli 91 | GURDJIAN D P M |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes Dokument